# EUROPÄISCHE PATENTSCHRIFT

(11) **EP 1 509 258 B1**
(45) Veröffentlichungstag und Bekanntmachung des Hinweises auf die Patenterteilung: **28.05.2008**
(21) Anmeldenummer: 03724768.1
(22) Anmeldetag: 02.06.2003
(51) Int. Cl.: A61M 1/00

(54) **THORAX DRAINAGE SYSTEM**
THORAX DRAINAGE SYSTEM
SYSTEME DE DRAINAGE THORACIQUE

(30) Priorität: 05.06.2002 CH 949022002
(43) Veröffentlichungstag der Anmeldung: 02.03.2005
(73) Patentinhaber: Medela Holding AG, 6340 Baar (CH)
(72) Erfinder: RÖLLIN, Richard, CH-6313 Menzingen (CH); MOSER, Beat, CH-5643 Sins (CH); KOCH, Urs, CH-6404 Greppen (CH)
(74) Vertreter: Clerc, Natalia
(86) Internationale Anmeldenummer: PCT/CH2003/000342
(87) Internationale Veröffentlichungsnummer: WO 2003/103747

(56) Entgegenhaltungen:
- US-A- 3 625 216
- US-A- 5 776 119

## Beschreibung

Die vorliegende Erfindung betrifft ein Thorax Drainage System nach dem Oberbegriff des Anspruchs 1. Die heute bekannten Systeme, welche alle mit vakuumabsaugung arbeiten, sind so gebaut, dass die meisten, wenn nicht alle, Systemkomponenten (mit Ausnahme der Vakuumpumpe) nach einmaligem Gebrauch weggeworfen werden. Ein weiterer Nachteil liegt darin, dass das Drainage System netzabhängig ist, somit der Patient bei Betrieb des Systems praktisch an sein Bett gebunden ist.

Das Thorax Drainage System ist für den Patienten von vitaler Bedeutung, da nach Eingriffen im Thoraxbereich im Thorax ein Vakuum aufgebaut bzw. aufrechterhalten werden muss, damit die Lunge sich in die normale "Betriebsgrösse" aufweiten kann.

Ein Thorax Drainage System der eingangs genannten Art ist aus US-A-3 625 216 bekannt.

US-A-5 776 119 offenbart eine Absaugvorrichtung mit einer batteriebetriebenen Vakuumpumpe.

Aufgabe der vorliegenden Erfindung war es, ein Thorax Drainage System zu schaffen, welches einerseits die medizinischen Anforderungen erfüllt und andererseits dem Patienten eine verbesserte Mobilität verschafft (u.a. um die Liegezeiten zu reduzieren). Gleichzeitig soll das System für ein und denselben Patienten mehrfach verwendet werden können (Kostenreduktion durch Mehrfachverwendung auch des Sekretsammelbehälters).

Diese Aufgabe wird beim vorliegenden Thorax Drainage System erfindungsgemäss durch die Merkmale des kennzeichnenden Teil von Anspruch 1 gelöst.

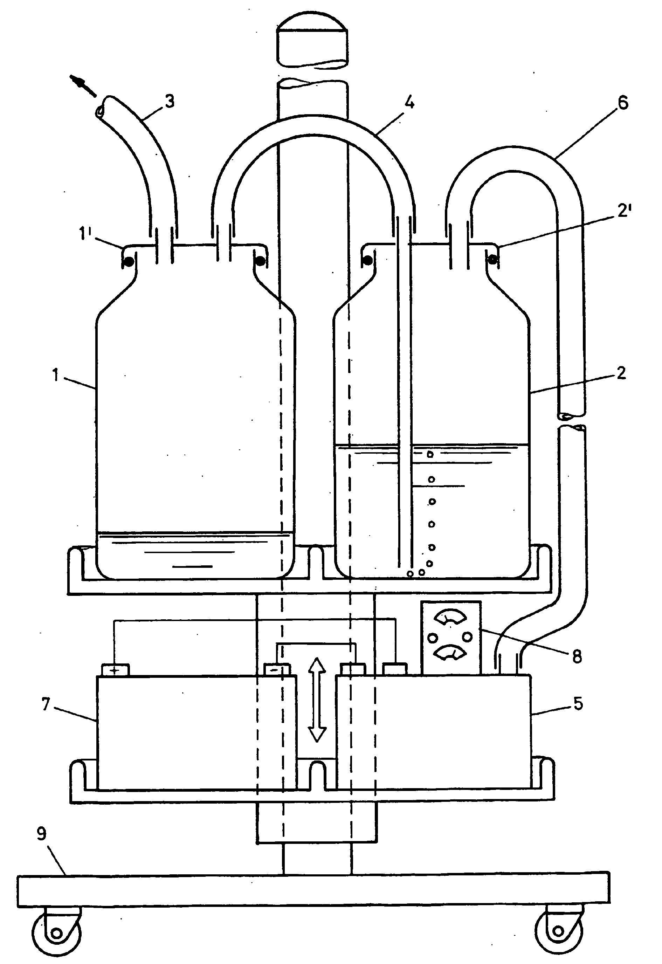

Ein Ausführungsbeispiel des Erfindungsgegenstandes wird nachstehend anhand der Zeichnung noch etwas näher erläutert. Die einzige Figur zeigt rein schematisch den Aufbau des erfindungsgemässen Systems:

Das die Sekretsammelkammer 1 bildende Behältnis ist mit einem Deckel 1' dicht verschlossen. Die zum Patienten führende Drainageleitung 3, als Wegwerfleitung ausgebildet, führt durch den Deckel 1' in die Kammer 1. Ein zweites Behältnis 2 bildet ein Unterwasserschloss und ist über die Verbindungsleitung 4 mit dem Behältnis 1 verbunden. Das teilweise mit Wasser gefüllte Unterwasserschloss bildet eine Art Rückschlagventil, bei welchem Luft oder Flüssigkeit abgeleitet aber nicht wieder zum Patienten zurückfliessen kann.

Das Behältnis 2, über den Deckel 2' ebenfalls dicht verschlossen, ist über eine Leitung 6 mit einer Vakuumpumpe 5 verbunden, welche ihrerseits von einer Batterie 7 betrieben wird (für den stationären Einsatz kann ein Netzanschluss wahlweise zuschaltbar sein). Für den Betrieb des Systems ist ein Steuergerät 8 vorgesehen.

Zur Verbesserung der Mobilität eines Patienten sind sämtliche Systemkomponenten auf einem verfahrbaren Gestell angeordnet, sodass dank dem Batteriebetrieb völlige Unabhängigkeit gewährleistet ist. Das Gestell kann selbstverständlich auch Infusionsbehältnisse zusätzlich aufnehmen.

Von Zeit zu Zeit auszuwechseln ist die Drainageleitung 3 (Wegwerfartikel) und das Behältnis 1 (Sekretsammelkamcner), welche gereinigt werden kann.

## Patentansprüche

1. Thorax Drainage System, enthaltend:
- ein erstes Behältnis (1), welches eine Sekretsammelkammer bildet,
- ein separates zweites Behältnis (2), welches ein Rückschlagventil in Form eines Unterwasserschlosses bildet,
- eine an einen Patienten anschliessbare Drainageleitung (3), welche in die Sekretsammelkammer (1) führt,
- eine erste Verbindungsleitung (4) zwischen den beiden Behältnissen (1, 2),
- eine Vakuumpumpe (5),
- eine zweite Verbindungsleitung (6) zwischen dem das Unterwasserschloss bildenden Behältnis (2) und der Vakuumpumpe (5) und
- ein verfahrbares Gestell (9) zur Aufnahme der beiden Behältnisse (1, 2),
**dadurch gekennzeichnet, dass**
- eine Batterie (7) zum netzunabhängigen Betrieb der Vakuumpumpe (5) auf dem verfahrbaren Gestell (9) angeordnet ist, wobei das verfahrbare Gestell (9) zur Aufnahme weiterer Systemkomponenten (1-8) vorgesehen ist, und
- die beiden Behältnisse (1, 2) mit zugehörigen Deckeln (1', 2'), in welche die erste und die zweite Verbindungsleitung (4, 6) und die Drainageleitung (3) münden, dicht verschliessbar sind.

2. Thorax Drainage System nach Anspruch 1, wobei die beiden separaten Behältnisse (1, 2) Mehrweg-Behältnisse sind.

3. Thorax Drainage System nach Anspruch 1 oder 2, wobei mindestens die Drainageleitung (3) eine Wegwerfleitung ist.

4. Thorax Drainage System nach einem der Ansprüche 1 bis 3, wobei das verfahrbare Gestell (9) zusätzlich zur Aufnahme von Infusionsbehältnissen vorgesehen ist.

## Claims

1. Thorax drainage system, comprising:
- a first container (1) forming a secretion-collecting chamber,
- a separate second container (2) forming a nonreturn valve in the form of an underwater seal,
- a drainage conduit (3) which can be attached to a patient and leads into the secretion-collecting chamber (1),
- a first connecting conduit (4) between the two containers (1, 2),
- a vacuum pump (5),
- a second connecting conduit (6) between the vacuum pump (5) and the container (2) forming the underwater seal and
- a mobile trolley (9) for receiving the two containers (1, 2),
**characterized in that**
- a battery (7) for mains-free operation of the vacuum pump (5) is arranged on the mobile trolley (9) and wherein the mobile trolley (9) is provided to receive further system components (1 - 8), and
- the two containers (1, 2) can be closed tight with associated covers (1', 2') into which the first and second connecting conduits (4, 6) and the drainage conduit (3) open.

2. Thorax drainage system according to Claim 1, in which the two separate containers (1, 2) are reusable containers.

3. Thorax drainage system according to Claim 1 or 2, in which at least the drainage conduit (3) is a disposable conduit.

4. Thorax drainage system according to one of Claims 1 to 3, in which the mobile trolley (9) is additionally provided to receive infusion containers.

## Revendications

1. Système de drainage thoracique, comprenant :
- un premier récipient (1), formant une chambre de collecte des sécrétions,
- un deuxième récipient séparé (2), qui forme une valve antiretour en forme de chambre de scellé-sous-eau,
- une conduite de drainage (3) pouvant être raccordée au patient, qui conduit dans la chambre de collecte des sécrétions (1).,
- une première conduite de connexion (4) entre les deux récipients (1, 2),
- une pompe à vide (5),
- une deuxième conduite de connexion (6) entre le récipient (2) formant la chambre de scellé-sous-eau et la pompe à vide (5) et
- un bâti déplaçable (9) pour recevoir les deux récipients (1, 2),
**caractérisé en ce que**
- une batterie (7) permettant un fonctionnement indépendant du réseau de la pompe à vide (5) est disposée sur le bâti déplaçable (9), le bâti déplaçable (9) étant prévu pour recevoir d'autres composants du système (1-8), et
- les deux récipients (1, 2) peuvent être fermés hermétiquement avec des couvercles associés (1', 2'), dans lesquels débouchent la première et la deuxième conduites de connexion (4, 6) et la conduite de drainage (3).

2. Système de drainage thoracique selon la revendication 1, dans lequel les deux récipients séparés (1, 2) sont des récipients à plusieurs voies.

3. Système de drainage thoracique selon la revendication 1 ou 2, dans lequel au moins la conduite de drainage (3) est une conduite jetable.

4. Système de drainage thoracique selon l'une quelconque des revendications 1 à 3, dans lequel le bâti déplaçable (9) est en outre prévu pour recevoir des récipients de transfusion.
